(19) Europäisches Patentamt
European Patent Office
Office européen des brevets

(11) EP 1 370 529 B1

(12) # EUROPÄISCHE PATENTSCHRIFT

(45) Veröffentlichungstag und Bekanntmachung des Hinweises auf die Patenterteilung:
**11.01.2006 Patentblatt 2006/02**

(21) Anmeldenummer: **02750542.9**

(22) Anmeldetag: **13.03.2002**

(51) Int Cl.:
***C07D 211/48*** *(2006.01)* ***C07D 211/52*** *(2006.01)*
***A61K 31/451*** *(2006.01)*

(86) Internationale Anmeldenummer:
**PCT/EP2002/002723**

(87) Internationale Veröffentlichungsnummer:
**WO 2002/072550 (19.09.2002 Gazette 2002/38)**

(54) **SUBSTITUIERTE DIMETHYL 1-(1-PHENYL-CYCLOHEXYL)-PIPERIDIN-3-YLMETHYL!-AMINE UND VERWENDUNG ALS ANALGETIKA**

SUBSTITUTED DIMETHYL- 1-(1-PHENYL-CYCLOHEXYL)-PIPERIDIN-3-YL METHYL]-AMINES AND THE USE OF THE SAME AS ANALGESICS

DIMETHYL- 1-(1-PHENYL-CYCLOHEXYL)-PIPERIDINE-3-YL METHYL]-AMINES SUBSTITUEES ET LEUR UTILISATION EN TANT QU'ANALGESIQUES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **14.03.2001 DE 10112198**

(43) Veröffentlichungstag der Anmeldung:
**17.12.2003 Patentblatt 2003/51**

(73) Patentinhaber: **Grünenthal GmbH**
**52078 Aachen (DE)**

(72) Erfinder:
- **SATTLEGGER, Michael**
**53123 Bonn (DE)**
- **REISSMÜLLER, Elke**
**33617 Bielefeld (DE)**

(56) Entgegenhaltungen:
**WO-A-00/06545**

- **ITZHAK ET AL: "New analgesic drugs derived from phencyclidine" JOURNAL OF MEDICINAL CHEMISTRY, AMERICAN CHEMICAL SOCIETY. WASHINGTON, US, 1981, Seiten 496-499, XP002099476 ISSN: 0022-2623 in der Anmeldung erwähnt**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 370 529 B1

## Beschreibung

Die Erfindung betrifft substituierte Dimethyl-[1-(1-phenyl-cyclohexyl)-piperidin-3-ylmethyl]-amine, Verfahren zu deren Herstellung, Arzneimittel enthaltend diese Verbindungen sowie die Verwendung dieser Stoffe zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind. So sind beispielsweise aus J. Med. Chem. 1981, 24, 496 -499 und Arzneim.-Forsch./Drug Res. 44 (II), Nr. 10 (1994), 1141 - 1144 Phencyclidin-Derivate mit analgetischer Wirkung bekannt. WO 0006545 offenbart strukturell ähnliche Piperidine, die sich unter anderem zur Schmerzbehandlung eignen.

Klassische Opioide, wie z.B. das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation, sowie eine Toleranzentwicklung auf. Sie sind außerdem bei neuropathischen oder inzidentiellen Schmerzen, wie sie insbesondere bei Tumorpatienten häufig auftreten, weniger wirksam.

Tramadolhydrochlorid - (1 RS,2RS)-2-[(Dimethylamino)methyl]-1-(3-methoxyphenyl)-cyclohexanol - nimmt unter den zentral wirksamen Analgetika eine Sonderstellung ein, da dieser Wirkstoff eine starke Schmerzhemmung ohne die für Opioide bekannten Nebenwirkungen hervorruft. (J. Pharmacol. Exptl. Ther. 267, 33 (1993)).

Die der Erfindung zugrundeliegende Aufgabe bestand in dem zur Verfügung stellen von analgetisch wirksamen Substanzen, die sich zu der Behandlung von starken Schmerzen, insbesondere zu der Behandlung von chronischen und neuropathischen Schmerzen, eignen. Darüber hinaus sollten diese Wirkstoffe möglichst wenig Nebenwirkungen der Opioid-Analgetika wie z.B. Übelkeit, Erbrechen, Abhängigkeit, Atemdepression, Obstipation aufweisen.

Erfindungsgemäß wird dies durch substituierte Dimethyl-[1-(1-phenylcyclohexyl)-piperidin-3-ylmethyl]-amine der allgemeinen Formel I erreicht, wobei diese Verbindungen eine ausgeprägte analgetische Wirkung aufweisen.

Gegenstand der Erfindung sind daher substituierte Dimethyl-[1-(1-phenylcyclohexyl)-piperidin-3-ylmethyl]-amine der allgemeinen Formel 1

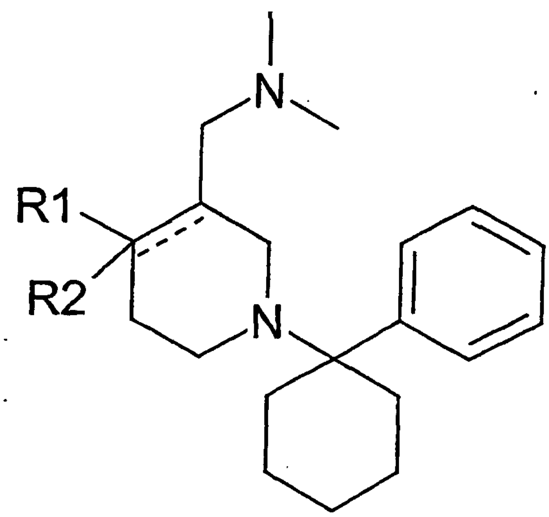

I

worin

R1 = H, $C_{1-12}$-Alkyl (verzweigt, unverzweigt), Vinyl, Phenyl (einfach oder vielfach substituiert mit $C_{1-5}$-Alkyl (verzweigt, unverzweigt), H, F, Cl, Br, OMe, OEt, OPr, OBu, SMe, OH und/oder $CF_3$),
Benzyl (einfach oder vielfach substituiert mit $C_{1-5}$-Alkyl (verzweigt, unverzweigt), H, F, Cl, Br, OMe, OEt, OPr, OBu, SMe, OH und/oder $CF_3$),
Phenethyl (einfach oder vielfach substituiert mit $C_{1-5}$-Alkyl (verzweigt, unverzweigt), H, F, Cl, Br, OMe, OEt,

OPr, OBu, SMe, OH und/oder $CF_3$) oder
Naphthyl (einfach oder vielfach substituiert mit $C_{1-5}$-Alkyl (verzweigt, unverzweigt), H, F, Cl, Br, OMe, OEt, OPr, OBu, OBz, SMe, OH und/oder $CF_3$), und

R2 = H, F, Cl, Br, OMe, OEt, OPr, OBu, OBz, SMe, OH, $CF_3$ oder Bindung zur Doppelbindung,
und/oder deren Enantiomere, Diastereomere, Basen oder Salze von physiologisch verträglichen Säuren.
Besonders bevorzugt sind folgende substituierte Dimethyl-[1-(1-phenylcyclohexyl)-piperidin-3-ylmethyl]-amine:
3-Dimethylaminomethyl-4-methyl-1-(1-phenyl-cyclohexyl)-piperidin-4-ol oder das entsprechende Dihydrochlorid
3-Dimethylaminomethyl-4-ethyl-1-(1-phenyl-cyclohexyl)-piperidin-4-ol oder das entsprechende Dihydrochlorid
3-Dimethylaminomethyl-1-(1-phenyl-cyclohexyl)-4-vinyl-piperidin-4-ol oder das entsprechende Dihydrochlorid
4-Butyl-3-dimethylaminomethyl-1-(1-phenyl-cyclohexyl)-piperidin-4-ol oder das entsprechende Dihydrochlorid
3-Dimethylaminomethyl-4-octyl-1-(1-phenyl-cyclohexyl)-piperidin-4-ol oder das entsprechende Dihydrochlorid
3-Dimethylaminomethyl-4-(3-methoxy-phenyl)-1-(1-phenyl-cyclohexyl)-piperidin-4-ol oder das entsprechende Dihydrochlorid
3-Dimethylaminomethyl-4-(2-fluoro-phenyl)-1-(1-phenyl-cyclohexyl)-piperidin-4-ol oder das entsprechende Dihydrochlorid
4-(3-Chloro-phenyl)-3-dimethylaminomethyl-1-(1-phenyl-cyclohexyl)-piperidin-4-ol oder das entsprechende Dihydrochlorid
4-Benzyl-3-dimethylaminomethyl-1-(1-phenyl-cyclohexyl)-piperidin-4-ol oder das entsprechende Dihydrochlorid
3-Dimethylaminomethyl-4-phenethyl-1-(1-phenyl-cyclohexyl)-piperidin-4-ol oder das entsprechende Dihydrochlorid
3-Dimethylaminomethyl-4-(3-hydroxy-phenyl)-1-(1-phenyl-cyclohexyl)-piperidin-4-ol oder das entsprechende Dihydrochlorid.

Ein weiterer Gegenstand der Erfindung ist ein Verfahren zur Herstellung von Dimethyl-[1-(1-phenyl-cyclohexyl)-piperidin-3-ylmethyl]-aminen der allgemeinen Formel I, die in R1 und R2 unterschiedlich substituiert sein können. Wenn entweder nur R1 oder R2 vorhanden ist, befindet sich eine Doppelbindung im Piperidinring zwischen Position 3 und 4.

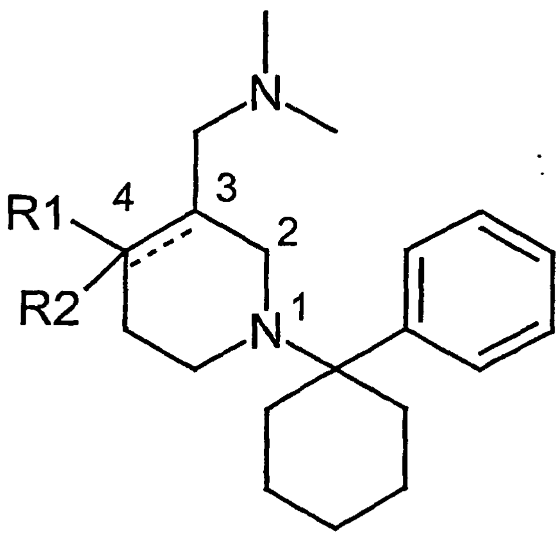

I

Die Herstellung der erfindungsgemäßen Verbindungen erfolgt in folgenden Schritten:

Aus Cylohexanon **II** und 1,4-Dioxa-8-aza-spiro [4.5]decan **III** wird das Enamin **IV** gebildet.

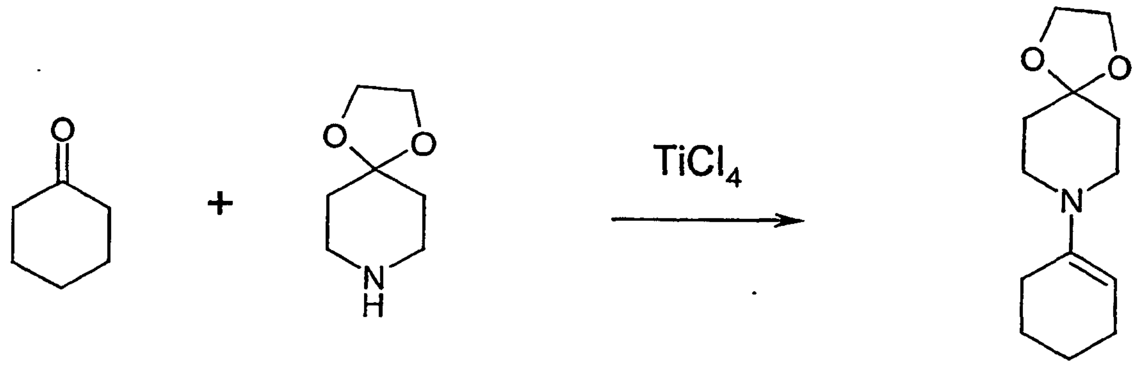


**II** **III** **IV**

Das Enamin **IV** wird direkt weiter mit Phenylmagnesiumchlorid **V** zum Amin **VI** umgesetzt:

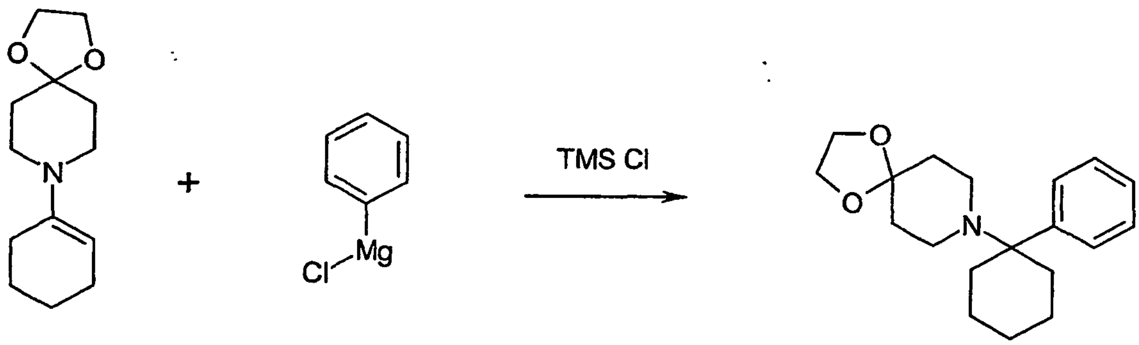


**IV** **V** **VI**

Das Amin **VI** wird in einem weiteren Schritt hydrolisiert und **VII** als Hydrochlorid gefällt.

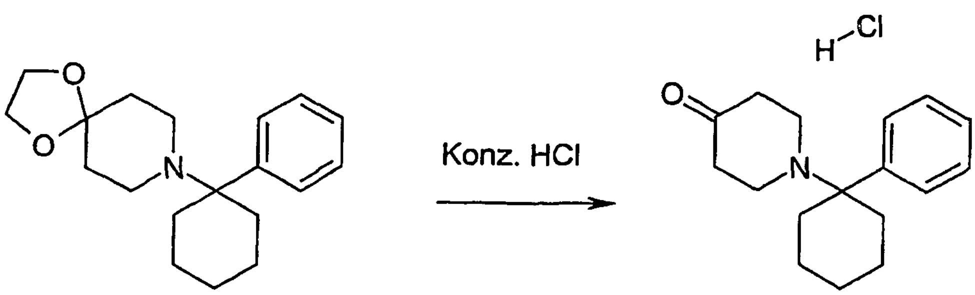


**VI** **VII**

Dieses Hydrochlorid **VII** wird mit einer Variante des Eschenmosersalzes **VIII** weiter zur Mannichbase **IX** umgesetzt.

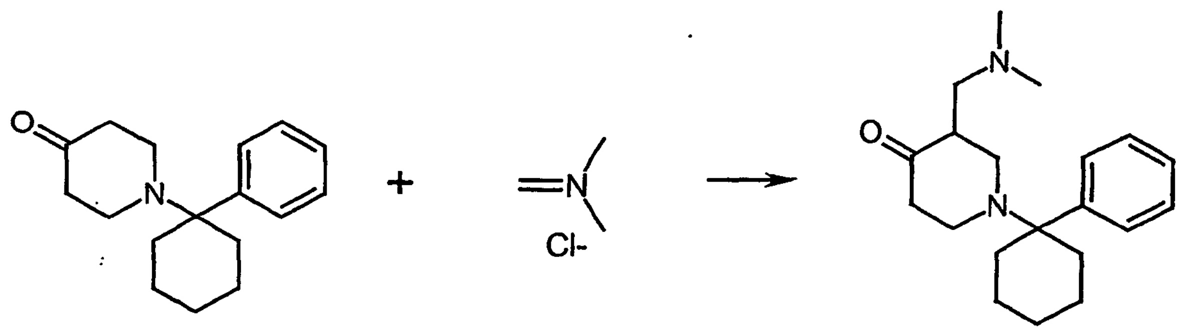

VII VIII IX

Dann erfolgt die Umsetzung mit einem Grignardreagenz **X** zu den Produkten **XI.**

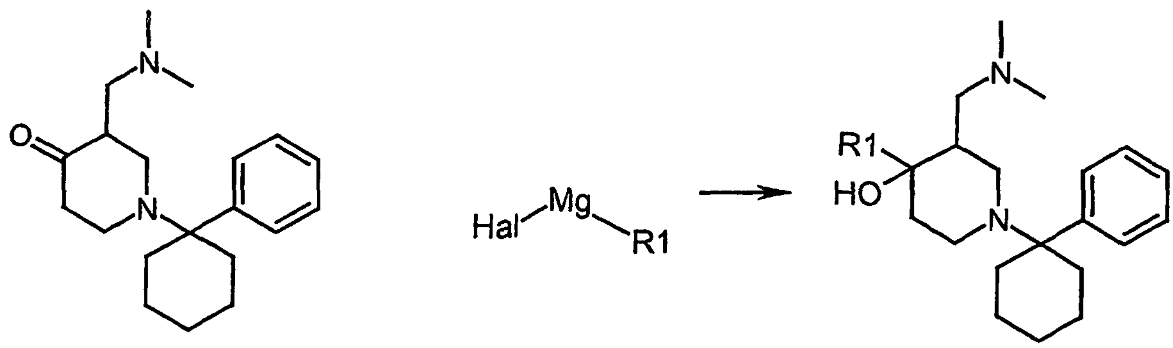

IX X XI

Die Verbindungen der allgemeinen Formel **XI** lassen sich weiter mit einer Reihe Reagenzien, die den oben definierten Rest R2 einbringen, insbesondere Halogenkohlenwasserstoffen, Ethern, Estern, Harnstoffen, Amiden, Carbonaten und verwandten Verbindungen zu den Verbindungen **XII** umsetzen.

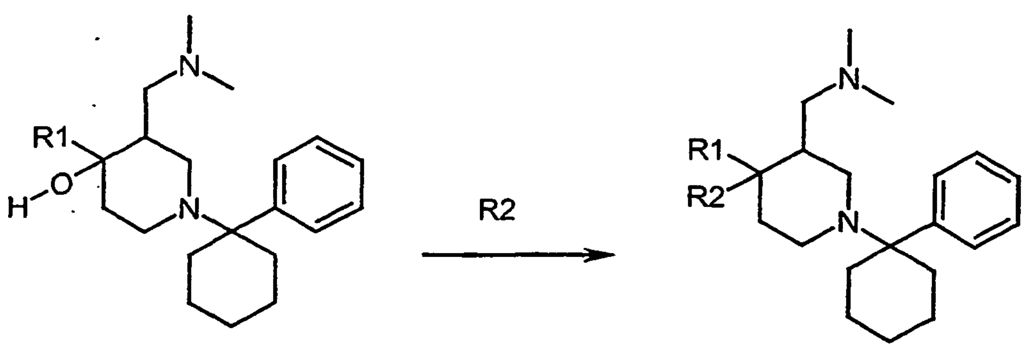


XI XII

Die erfindungsgemäßen Verbindungen lassen sich mit Bernsteinsäure, Weinsäure, Mandelsäure, Fumarsäure,

Milchsäure, Zitronensäure, Glutaminsäure und/oder Asparaginsäure, in an sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon durchgeführt. Zur

Herstellung der Hydrochloride eignet sich darüber hinaus Trimethylchlorsilan in Methylethylketon.

Die erfindungsgemäßen substituierten Dimethyl-[1-(1-phenyl-cyclohexyl)-piperidin-3-ylmethyl]-amine der allgemeinen Formel I sind toxikologisch unbedenklich und stellen daher geeignete pharmazeutische Wirkstoffe dar.

Ein weiterer Gegenstand der Erfindung sind daher Arzneimittel, die als Wirkstoff wenigstens ein substituiertes Dimethyl-[1-(1-phenyl-cyclohexyl)-piperidin-3-ylmethyl]-amin der allgemeinen Formel 1 und/oder deren Enantiomere, Diastereomere, Basen oder Salze von physiologisch verträglichen Säuren enthalten.

Bevorzugt enthalten sind dabei Enantiomerengemische des Wirkstoffs in nicht äquimolaren Mengen, wobei eines der Enantiomeren einen relativen Anteil von 5 bis 45 Massenprozent an dem Gemisch aufweist.

Die erfindungsgemäßen Arzneimittel eignen sich zur Bekämpfung von Schmerzen.

Ein weiterer Gegenstand der Erfindung ist daher auch die Verwendung von wenigstens einem substituierten Dimethyl-[1-(1-phenyl-cyclohexyl)-piperidin-3-ylmethyl]-amin der allgemeinen Formel I und/oder deren Enantiomere, Diastereomere, Basen oder Salze von physiologisch verträglichen Säuren zur Herstellung eines Arzneimittels zur Bekämpfung von Schmerzen.

Zur Zubereitung entsprechender pharmazeutischer Formulierungen werden neben mindestens einem substituierten Dimethyl-[1-(1-phenyl-cyclohexyl)-piperidin-3-ylmethyl]-amin der allgemeinen Formel I Trägermaterialien, Füllstoffe, Lösungsmittel, Verdünnungsmittel, Farbstoffe und/oder Bindemittel eingesetzt. Die Auswahl der Hilfsstoffe, sowie die einzusetzenden Mengen derselben hängt davon ab, ob das Arzneimittel oral, intravenös, intraperitoneal, intradermal, intramuskulär, intranasal, buccal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 50 bis 500 mg/kg wenigstens eines Dimethyl-[1-(1-phenyt-cyclohexyl)-piperidin-3-ylmethyl]-amins der allgemeinen Formel I appliziert.

## Beispiele

### Allgemeine Bemerkungen

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Schmelztemperaturen sind unkorrigiert.

Soweit nicht anders angegeben, wurde Petrolether mit dem Siedebereich von 50 bis 70 °C, benutzt. Die Angabe Ether bedeutet Diethylether.

Als stationäre Phase für die Säulenchromatographie wurde Kieselgel 60 (0,040 bis 0,063 mmm) der Firma E. Merck, Darmstadt, eingesetzt.

Die dünnschicht-chromatographischen Untersuchungen wurden mit HPLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in VolumenNolumen angegeben.

### Beispiel 1

3-Dimethylaminomethyl-4-methyl-1-(1-phenyl-cyclohexyl)-piperidin-4-ol Dihydrochlorid (1)

### 1.Stufe

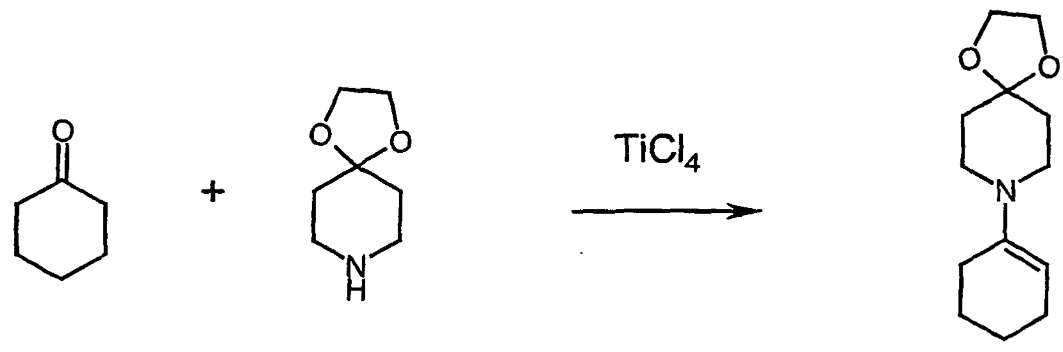


II III IV

Zur Darstellung von 8-Cyclohex-1-enyl-1,4-dioxa-8-aza-spiro[4.5]decan **IV** wurden 54 ml (0,5 mol) Cylohexanon mit 200 ml (1,5 mol) 1,4-Dioxa-8-azaspiro [4.5]decane **III** in 0,5 l Diethylether gelöst und eine halbe Stunde gerührt. Dann wurden 31 ml Titantetrachlorid in 0,5 l n-Hexan bei 0°C innerhalb von 60 min zugetropft. Nach beendeter Zugabe wurde der Ansatz langsam auf 20 °C erwärmt und 24 h nachgerührt. Der entstandene Niederschlag wurde abgesaugt und verworfen. Das Filtrat wurde eingeengt und direkt weiter umgesetzt. Die Ausbeute betrug 83 g (0,37 mol, 71 %).

**2. Stufe**

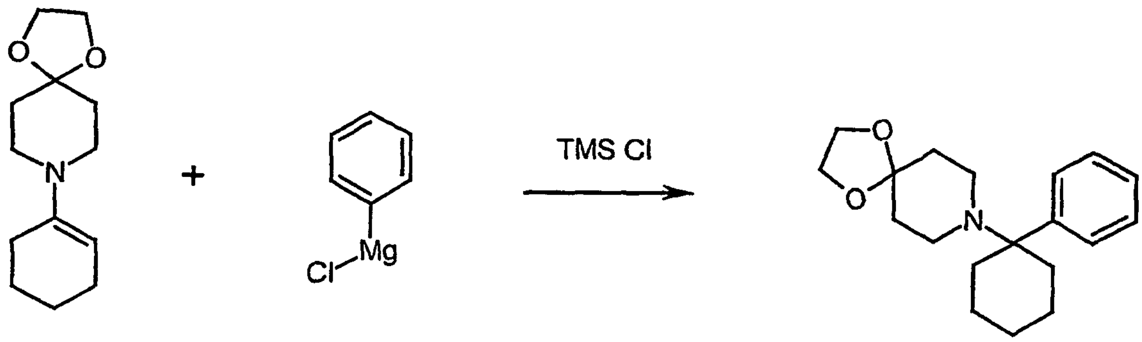


IV V VI

83 g (0,37 mol) 8-Cyclohex-1-enyl-1,4-dioxa-8-aza-spiro [4.5]decan **IV** wurden mit 200 ml 2M Phenylmagnesiumchlorid-lösung **V** umgesetzt. Dazu wurden 52 ml Trimethylchlorsilan in 0,75 l Methylenchlorid mit 2 ml Wasser vorgelegt und das Enamin **IV** zugetropft. Dann wurde unter Eisbadkühlung das Grignardreagenz zugegeben und 3 h nachgerührt. Es wurde mit 200 ml Ammoniumchloridlösung hydrolisiert und die wässrige Phase mit 0,5 1 Methylenchlorid extrahiert. Das Produkt **VI** wurde säulenchromatographisch auf Kieselgel mit Diisopropylether gereinigt. Die Ausbeute betrug 36 g (0,12 mol, 32 %).

**3.Stufe**

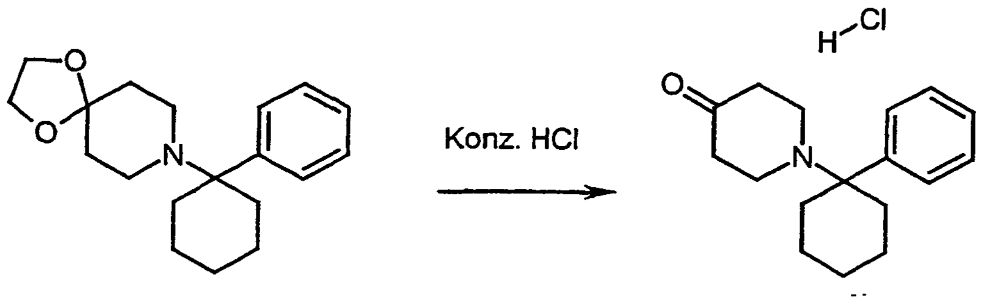


VI VII

Das Amin **VI** wurde in einem weiteren Schritt hydrolisiert und als Hydrochlorid **VII** gefällt. Dafür wurden 36 g (0,12 mol) **VI** mit 250 ml konzentrierter HCl bei 20 °C versetzt und 12 h gerührt. Es wurde mit ammoniakalischer Lösung alkalisiert und mit Diethylether extrahiert. Die freie Base wurde mit Trimethylchlorsilan als Hydrochlorid gefällt. Die Ausbeute betrug 21 g (0,072 mol, 60 %).

**4.Stufe**

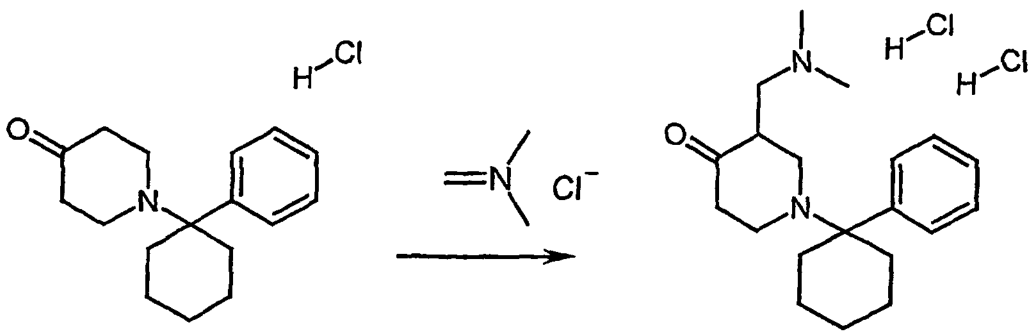

VII VIII IX

Das Hydrochlorid **VII** wurde mit einer Variante des Eschenmosersalzes **VIII** zur Mannichbase **IX** umgesetzt. Dabei wurden 6,0 g (20 mmol)der Verbindung **VII** mit 2,1 g (22 mmol) der Verbindung **VIII** in 50 ml trockenem Tetrahydrofuran 48 h bei 20°C gerührt. Anschließend wurde der Reaktionsansatz in eine basische, wässrige ammoniakalische Lösung gegossen und die freie Base von **IX** mit 3 **X** 100 ml Methylenchlorid extrahiert. Die organischen Phasen wurden vereinigt, getrocknet und das Lösungmittel im Vakuum entfernt. Der Rückstand wurde in 50 ml Methylethylketon gelöst und mit 5 ml Trimethylsilylchlorid versetzt. Es wurden 3,0 g (8 mmol, 40 %) der Verbindung **IX** erhalten.

**5. Stufe**

Nach der Freisetzung des Hydrochlorides **IX** in die freie Base, erfolgte die Umsetzung von **IX** mit einem Methylmagnesiumchlorid **X** zu **XI** und nach Fällung als Dihydrochlorid zu **(1).**

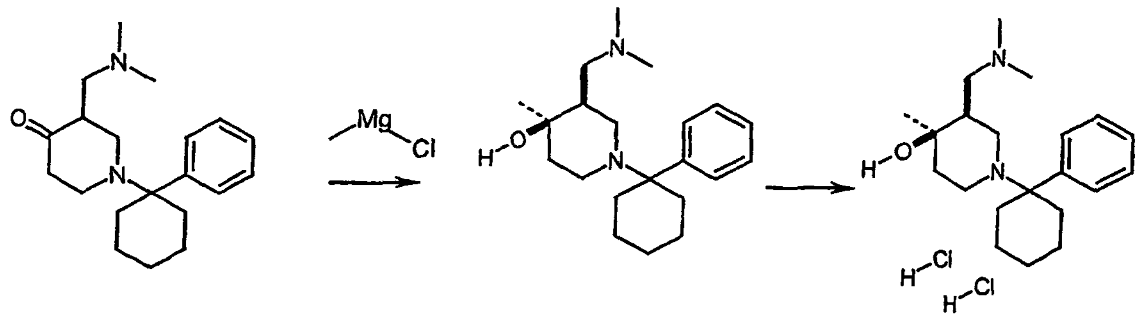

**IX** **X** **XI** **1**

Dazu wurden 0,57 g (1,8 mmol) **IX** in 2,5 ml Tetrahydrofuran gelöst und auf -20°C abgekühlt. Unter Schutzgas wurden 1,0 ml (3 mmol) 3M Methylmagnesiumchloridlösung in THF zugegeben und über Nacht gerührt. Anschließend wurden 2 ml Ammoniumchloridlösung zur Hydrolyse zugegeben und die wässrige Phase mit Ether extrahiert. Nach der Fällung als Dihydrochlorid betrug die Ausbeute von (1) 0,2 g (0,3 mmol, 16%). Der Zersetzungspunkt der Verbindung (**1**) lag bei 220°C.

**Beispiel 2**

3-Dimethylaminomethyl-4-ethyl-1-(1-phenyl-cyclohexyl)-piperidin-4-ol Dihydrochlorid (**2**)

Die Synthesevorschriften sind unter **Beispiel 1** beschrieben. Anstelle von Methylmagnesiumchlorid wurde Ethylmagnesiumchlorid verwendet. Bei der Umsetzung von 0,57 g (1,8 mmol) **IX** betrug die Ausbeute von (2) 21 mg (0,036 mmol, 2 %). Die Verbindung zersetzte sich ab 190°C.

**Beispiel 3**

3-Dimethylaminomethyl-4-vinyl-1-(1-phenyl-cyclohexyl)-piperidin-4-ol Dihydrochlorid (**3**)

Die Synthesevorschriften sind unter **Beispiel 1** beschrieben. Anstelle von Methylmagnesiumchlorid wurde Vinylmagnesiumchlorid verwendet. Bei der Umsetzung von 0,57 g (1,8 mmol) **IX** betrug die Ausbeute von (3) 37 mg (0,09 mmol, 3 %). Die Verbindung zersetzte sich ab 190°C

**Beispiel 4**

4-Butyl-3-dimethylaminomethyl-1-(1-phenyl-cyclohexyl)-piperidin-4-ol Dihydrochlorid **(4)**

Die Synthesevorschriften sind unter **Beispiel 1** beschrieben. Anstelle von Methylmagnesiumchlorid wurde Butylmagnesiumchlorid verwendet. Bei der Umsetzung von 0,57 g (1,8 mmol) **IX** betrug die Ausbeute von **(4)** 37 mg (0,09 mmol, 3 %). Der Schmelzpunkt der Verbindung betrug 225°C.

**Beispiel 5**

4-Ocytl-3-dimethylaminomethyl-1-(1-phenyl-cyclohexyl)-piperidin-4-ol Dihydrochlorid **(5)**

Die Synthesevorschriften sind unter **Beispiel 1** beschrieben. Anstelle von Methylmagnesiumchlorid wurde Octylmagnesiumchlorid verwendet. Bei der Umsetzung von 0,57 g (1,8 mmol) **IX** betrug die Ausbeute von (**5**) 104 mg (0,22 mmol, 12 %).

**Beispiel 6**

3-Dimethylaminomethyl-4-(3-methoxy-phenyl)-1-(1-phenyl-cyclohexyl)-piperidin-4-ol Dihydrochlorid (**6**)

Die Synthesevorschriften sind unter **Beispiel 1** beschrieben. Anstelle von Methylmagnesiumchlorid wurde 3-Methoxy-phenylmagnesiumbromid verwendet. Bei der Umsetzung von 0,57 g (1,8 mmol) **IX** betrug die Ausbeute von **(6)** 491 mg (1,07 mmol, 60%). Der Schmelzpunkt der Verbindung betrug 245 °C.

**Beispiel 7**

3-Dimethylaminomethyl-4-(2-fluoro-phenyl)-1-(1-phenyl-cyclohexyl)-piperidin-4-ol Dihydrochlorid **(7)**

Die Synthesevorschriften sind unter **Beispiel 1** beschrieben. Anstelle von Methylmagnesiumchlorid wurde 2-Fluoro-phenylmagnesiumjodid verwendet. Bei der Umsetzung von 0,57 g (1,8 mmol) **IX** betrug die Ausbeute von (7) 267 mg (0,55 mmol, 31%). Die Verbindung zersetzte sich ab 96°C.

**Beispiel 8**

3-Dimethylaminomethyl-4-(3-chloro-phenyl)-1-(1-phenyl-cyclohexyl)-piperidin-4-ol Dihydrochlorid **(8)**

Die Synthesevorschriften sind unter **Beispiel 1** beschrieben. Anstelle von Methylmagnesiumchlorid wurde 3-Chloro-phenylmagnesiumjodid verwendet. Bei der Umsetzung von 0,57 g (1,8 mmol) **IX** betrug die Ausbeute von (8) 177 mg (0,35 mmol, 19%). Die Verbindung zersetzte sich ab 96°C.

**Beispiel 9**

3-Dimethylaminomethyl-4-(benzyl)-1-(1-phenyl-cyclohexyl)-piperidin-4-ol Dihydrochlorid (**9**)

Die Synthesevorschriften sind unter **Beispiel 1** beschrieben. Anstelle von Methylmagnesiumchlorid wurde Benzylmagnesiumchlorid verwendet. Bei der Umsetzung von 0,57 g (1,8 mmol) **IX** betrug die Ausbeute von **(9)** 213 mg (0,48 mmol, 27%).

**Beispiel 10**

3-Dimethylaminomethyl-4-(phenethyl)-1-(1-phenyl-cyclohexyl)-piperidin-4-ol Dihydrochlorid (10)

Die Synthesevorschriften sind unter **Beispiel 1** beschrieben. Anstelle von Methylmagnesiumchlorid wurde Phenylmagnesiumbromid verwendet. Bei der Umsetzung von 0,57 g (1,8 mmol) **IX** betrug die Ausbeute von **(10)** 255 mg (0,56 mmol, 31 %). Die Verbindung zersetzte sich ab 234°C.

**Beispiel 11**

3-Dimethylaminomethyl-4-(3-hydroxy-phenyl)-1-(1-phenyl-cyclohexyl)-piperidin-4-ol Dihydrochlorid (**11**)

Die Synthesevorschriften sind bis zum letzten Schritt analog **Beispiel 6** beschrieben. Anschließend wurde der Methylether in das Phenol gespalten. Dazu wurden 0,5 g (1,2 mmol) der freien Base von Verbindung (6) in 5 ml Toluol gelöst und bei 0°C und Stickstoffatmosphäre mit 10 ml Dibutylaluminium-hydrid Lösung (1,5 M in Toluol) versetzt. Nach 12 h Rühren wurde der Ansatz mit 5 ml Ethylessigester und 5ml Ethanol hydrolisiert, das Lösungsmittel im Vakuum entfernt und der Rückstand in Methylethylketon gelöst und mit 1,0 ml Trimethylsilylchlorid versetzt. Die Ausbeute von (11) betrug 71 mg (0,15 mmol, 12%). Die Verbindung schmolz zwischen 245 und 248°C.

**Pharmakologische Untersuchungen**

**Writhing-Test an der Maus**

Die analgetische Wirksamkeit der erfindungsgemäßen Verbindungen im Phenylchinon-induzierten Writhing-Test, modifiziert nach I.C. Hendershot, J. Forsaith in J. Pharmacol. Exp. Ther. 125, 237 - 240 (1959), wurde an der Maus untersucht. Hierzu wurden männliche Mäuse mit einem Gewicht von 25 bis 30 g verwendet. Gruppen von

jeweils 10 Tieren pro Substanzdosis erhielten 10 Minuten nach intravenöser Gabe der Prüfsubstanzen 0,3 ml/Maus einer 0,02 %-igen wässrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden anschließend einzeln in Beobachtungskäfige gesetzt. Mit Hilfe eines Drucktastenzählers wurde die Anzahl der Schmerz-induzierten Streckbewegungen (sogenannte Writhing-Reaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung mit Phenylchinon erhielten.

Alle Substanzen wurden in der Standarddosis von 10 mg/kg getestet. Die prozentuale Hemmung (% Hemmung) der Writhingreaktionen durch eine Substanz wurde nach folgender Formel berechnet:

$$\%\ \text{Hemmung} = 100 - \left[\frac{\text{Writhingreaktion behandelte Tiere}}{\text{Writhingreaktion Kontrolle}} \times 100\right]$$

Alle untersuchten erfindungsgemäßen Verbindungen zeigten eine mittelstarke bis starke analgetische Wirkung.

Die Ergebnisse ausgewählter Writhing-Untersuchungen sind in der **Tabelle 1** zusammengefaßt.

**Tabelle 1:** Analgesieprüfung im Writhing-Test an der Maus

| Beispiel Nr. | % Hemmung der Writhing-Reaktionen 10 mg/kg i.v. |
|---|---|
| 1 | 33 |
| 5 | 31 |
| 6 | 54 |
| 8 | 40 |
| 9 | 35 |
| 11 | 73 |

**Formalin-Test, Maus**

Die Untersuchungen zur Bestimmung der antinociceptiven Wirkung der erfindungsgemäßen Verbindungen 6 und 11 wurden im Formalin-Test an männlichen Mäusen (NMRI, 20 bis 30 g) durchgeführt.

Im Formalin-Test werden die erste (frühe) Phase (0 bis 15 min nach Formalin-Injektion) und die zweite (späte) Phase (15 bis 60 min nach Formalin-Injektion) unterschieden (D. Dubuisson, S.G. Dennis, Pain 4, 161 -174 (1977)). Die frühe Phase stellt als direkte Reaktion auf die Formalin-Injektion ein Modell für Akutschmerz dar, während die späte Phase als Modell für persistierenden (chronischen) Schmerz angesehen wird (T.J. Coderre, J. Katz, A.L. Vaccarino, R. Melzack, Pain 52, 259 - 285 (1993)).

Die erfindungsgemäßen Verbindungen wurden in der zweiten Phase des Forrnalin-Tests untersucht, um Aussagen über Substanzwirkungen im chronisch/entzündlichen Schmerz zu erhalten.

Durch eine einmalige subkutane Formalin-Injektion (20 µl, 1 %-ig) in die dorsale Seite der rechten Hinterpfote wurde bei freibeweglichen Versuchstieren eine nociceptive Reaktion induziert, die sich in deutlichem Lecken und Beißen der betroffenen Pfote äußert.

Für den Untersuchungszeitraum von drei Minuten in der zweiten (späten) Phase des Formalin-Tests (21. bis 24. min nach Formalin-Applikation) wurde das nozizeptive Verhalten durch Beobachtung der Tiere kontinuierlich erfaßt. Die Quantifizierung des Schmerzverhaltens erfolgte durch Summation der Sekunden, in denen die Tiere in dem Untersuchungszeitraum Lecken und Beißen der betroffenen Pfote zeigten. Nach Injektion von Substanzen, die im Formalin-Test antinozizeptiv wirksam sind, sind die beschriebenen Verhaltensweisen der Tiere reduziert, evtl. sogar aufgehoben. Der Vergleich erfolgte mit Kontrolltieren, die Vehikel (Lösungsmittel) vor Formalinapplikation erhalten hatten. Basierend auf der Quantifizierung des Schmerzverhaltens wurde die Substanzwirkung im Formalin-Test als Änderung gegen Kontrolle in Prozent ermittelt. Abhängig von der Applikationsart der erfindungsgemäßen Verbindungen wurde der Applikationszeitpunkt vor der Formalin-Injektion gewählt (intravenös: 5 min).

Die Ergebnisse ausgewählter Untersuchungen im Formalin-Test Maus sind in der nachfolgenden Tabelle zusammengefaßt.

**Tabelle 2:** Antinozizeptive Wirkung der erfindungsgemäßen Verbindungen im Formalin-Test Maus

| Beisp. Nr. | % Hemmung gegen Kontrolle 10 mg/kg i.v. |
|---|---|
| 6 | 55 |
| 11 | 59 |

## Patentansprüche

1. Substituierte Dimethyl-[1-(1-phenyl-cyclohexyl)-piperidin-3-ylmethyl]-amine der allgemeinen Formel I

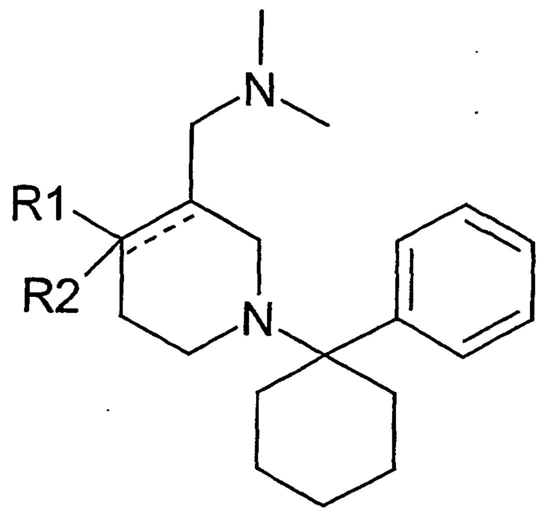

I

worin

R1 = H, $C_{1-12}$-Alkyl (verzweigt, unverzweigt), Vinyl, Phenyl (einfach oder vielfach substituiert mit $C_{1-5}$-Alkyl (verzweigt, unverzweigt), H, F, Cl, Br, OMe, OEt, OPr, OBu, SMe, OH, und/oder $CF_3$),
Benzyl (einfach oder vielfach substituiert mit $C_{1-5}$-Alkyl (verzweigt, unverzweigt), H, F, Cl, Br, OMe, OEt, OPr, OBu, SMe, OH, und/oder $CF_3$),
Phenethyl (einfach oder vielfach substituiert mit $C_{1-5}$-Alkyl (verzweigt, unverzweigt), H, F, Cl, Br, OMe, OEt, OPr, OBu, SMe, OH, und/oder $CF_3$), oder
Naphthyl (einfach oder vielfach substituiert mit $C_{1-5}$-Alkyl (verzweigt, unverzweigt), H, F, Cl, Br, OMe, OEt, OPr, OBu, OBz, SMe, OH, und/oder $CF_3$), und
R2 = H, F, Cl, Br, OMe, OEt, OPr, OBu, OBz, SMe, OH, $CF_3$ oder Bindung zur Doppelbindung,

und/oder deren Enantiomere, Diastereomere, Basen oder Salze von physiologisch verträglichen Säuren.

2. Verbindungen gemäß Anspruch 1, **dadurch gekennzeichnet, daß** R1 unverzweigtes $C_{1-8}$-Alkyl ist und R2 die Bedeutung gemäß Anspruch 1 hat.

3. Verbindungen gemäß Anspruch 1, **dadurch gekennzeichnet, daß** R1 Vinyl ist und R2 die Bedeutung gemäß Anspruch 1 hat.

4. Verbindungen gemäß Anspruch 1, **dadurch gekennzeichnet, daß** R1 ein mit F, Cl, OH, oder OMe substituierter Phenylrest ist und R2 die Bedeutung gemäß Anspruch 1 hat.

**5.** Verbindungen gemäß Anspruch 1, **dadurch gekennzeichnet, daß** R1 Benzyl ist und R2 die Bedeutung gemäß Anspruch 1 hat.

**6.** Verbindungen gemäß Anspruch 1, **dadurch gekennzeichnet, daß** R1 Phenethyl ist und R2 die Bedeutung gemäß Anspruch 1 hat.

**7.** Verbindungen gemäß Anspruch 1, **dadurch gekennzeichnet, daß** R2 OH ist und R1 die Bedeutung gemäß Anspruch 1 hat.

**8.** Verbindungen gemäß Anspruch 1:

3-Dimethylaminomethyl-4-methyl-1-(1-phenyl-cyclohexyl)-piperidin-4-ol oder das entsprechende Dihydrochlorid
3-Dimethylaminomethyl-4-ethyl-1-(1-phenyl-cyclohexyl)-piperid in-4-ol oder das entsprechende Dihydrochlorid
3-Dimethylaminomethyl-1-(1-phenyl-cyclohexyl)-4-vinyl-piperidin-4-ol oder das entsprechende Dihydrochlorid
4-Butyl-3-dimethylaminomethyl-1-(1-phenyl-cyclohexyl)-piperidin-4-ol oder das entsprechende Dihydrochlorid
3-Dimethylaminomethyl-4-octyl-1-(1-phenyl-cyclohexyl)-piperidin-4-ol oder das entsprechende Dihydrochlorid
3-Dimethylaminomethyl-4-(3-methoxy-phenyl)-1-(1-phenyl-cyclohexyl)-piperidin-4-ol oder das entsprechende Dihydrochlorid
3-Dimethylaminomethyl-4-(2-fluoro-phenyl)-1-(1-phenyl-cyclohexyl)-piperidin-4-ol oder das entsprechende Dihydrochlorid
4-(3-Chloro-phenyl)-3-dimethylaminomethyl-1-(1-phenyl-cyclohexyl)-piperidin-4-ol oder das entsprechende Dihydrochlorid
4-Benzyl-3-dimethylaminomethyl-1-(1-phenyl-cyclohexyl)-piperidin-4-ol oder das entsprechende Dihydrochlorid
3-Dimethylaminomethyl-4-phenethyl-1-(1-phenyl-cyclohexyl)-piperidin-4-ol oder das entsprechende Dihydrochlorid
3-Dimethylaminomethyl-4-(3-hydroxy-phenyl)-1-(1-phenyl-cyclohexyl)-piperidin-4-ol oder das entsprechende Dihydrochlorid

**9.** Verfahren zur Herstellung von substituierten Dimethyl-[1-(1-phenylcyclohexyl)-piperidin-3-ylmethyl]aminen der allgemeinen Formel I nach Anspruch 1, wobei man Cyclohexanon (Formel **II)** mit 1,4-Dioxa-8-azaspiro [4.5]decan (Formel **III)** in Gegenwart von Titantetrachlorid zum Enamin der Formel **IV**

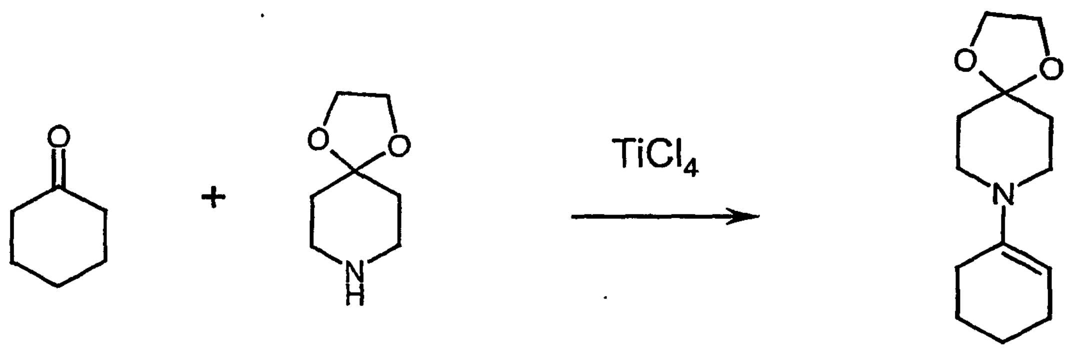


II III IV

umsetzt, welches weiter mit Phenylmagnesiumchlorid (Formel **V**) in Gegenwart von Trimethylchlorsilan zum Amin der Formel **VI**

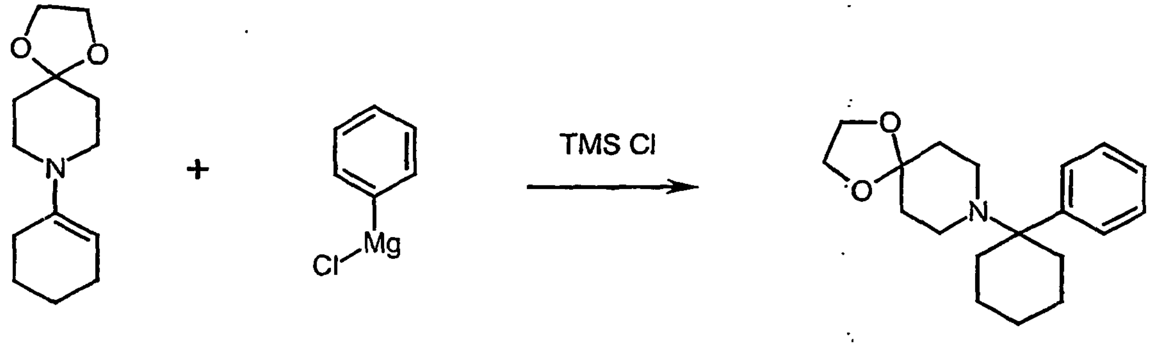


IV V VI

umgesetzt wird, anschließend das erhaltene Amin der Formel **VI** hydrolisiert und als Hydrochlorid der Formel **VII**

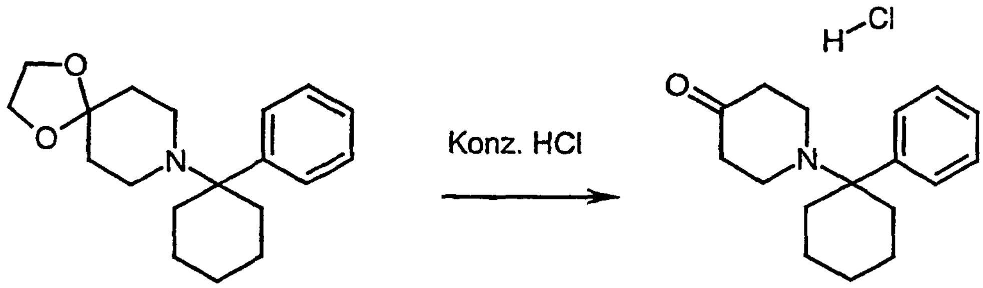


VI VII

gefällt wird, welches mit einer Variante des Eschenmosersalzes nach Formel **VIII** weiter zur Mannichbase der Formel **IX**

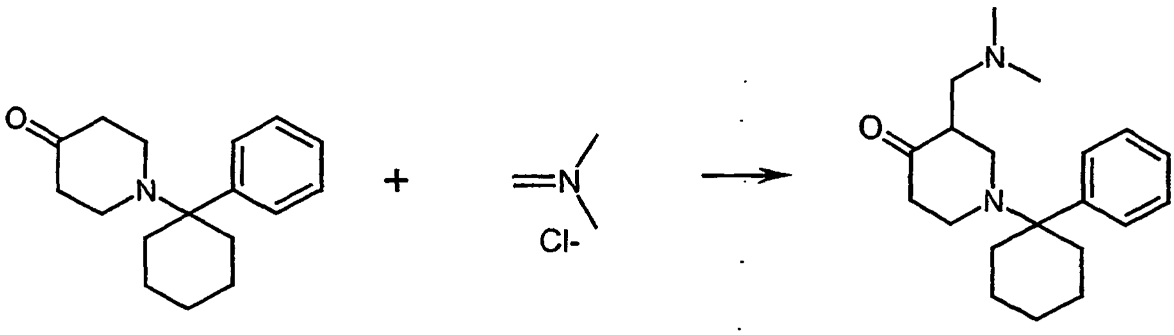

VII VIII IX

umgesetzt wird, wonach die Umsetzung der freien Base nach Formel **IX** mit einem Grignardreagenz der Formel **X,** das den organischen Rest R1 aufweist, zu den Verbindungen der Formel **XI**

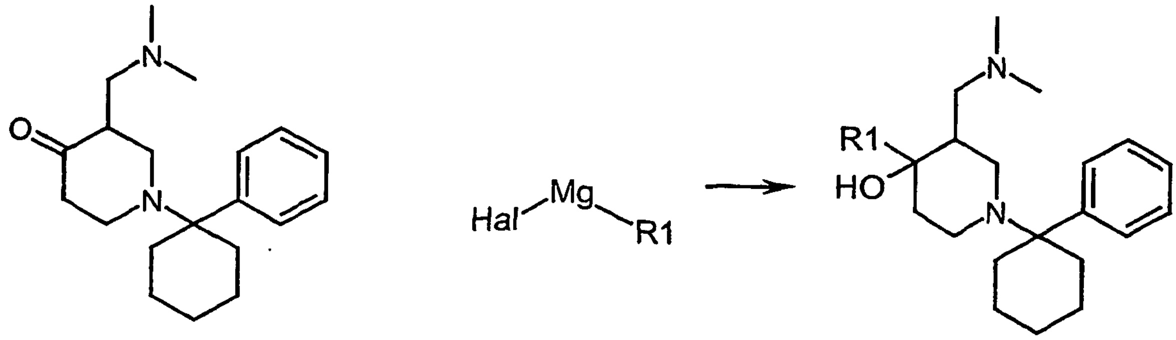

IX X XI

erfolgt, welche nach üblichen Methoden gereinigt und als Salze von physiologischen verträglichen Säuren isoliert werden, wobei man

- Verbindungen der Formel **XII** erhält, indem Verbindungen der Formel **XI** mit Reagenzien, die die OH-Gruppe in 4-Position der Verbindungen der Formel **XI** durch den oben definierten Rest R2, ausgenommen die OH-Gruppe, ersetzen, insbesondere Halogenkohlenwasserstoffen, Ethern, Estern, Harnstoffen, Amiden, Carbonaten und verwandten Verbindungen

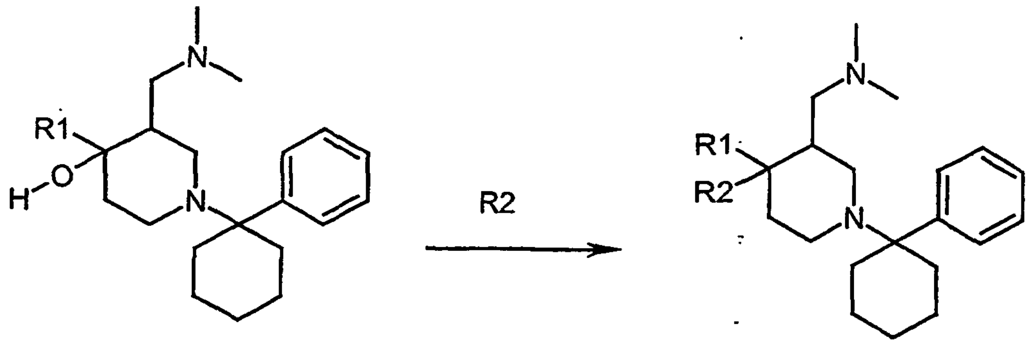


XI XII

umgesetzt werden,
- Verbindungen der Formel **XIII** erhält, indem Verbindungen der Formel **XI** dehydratisiert werden,

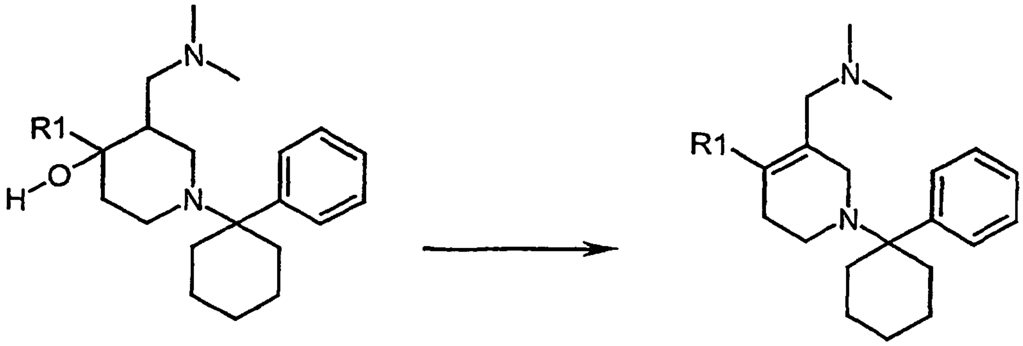

XI XIII

und

• Verbindungen der Formel **XIV** erhält, indem man Verbindungen der Formel **XIII** mit Wasserstoff

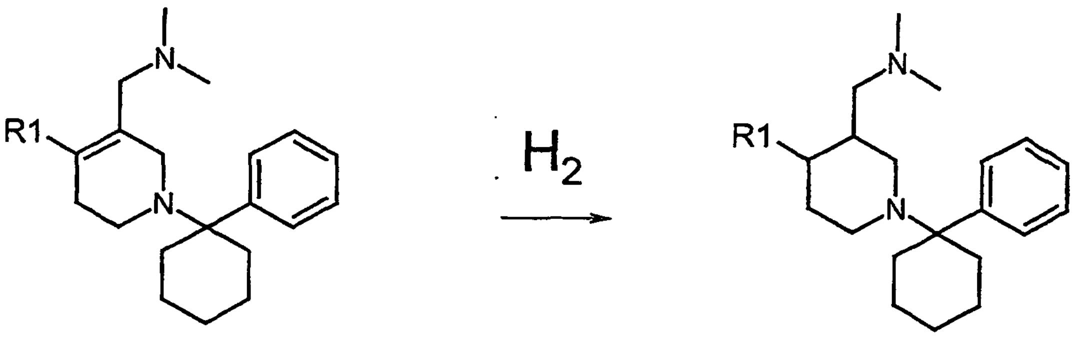


XIII XIV

reduziert.

**10.** Arzneimittel enthaltend als Wirkstoff wenigstens eine Verbindung der allgemeinen Formel **I** gemäß Anspruch 1 und/oder deren Enantiomere, Diastereomere, Basen oder Salze von physiologisch verträglichen Säuren.

**11.** Arzneimittel nach Anspruch 10 enthaltend als Wirkstoff ein Gemisch der Enantiomeren einer Verbindung der allgemeinen Formel 1 gemäß Anspruch 1, wobei die beiden Enantiomeren nicht in äquimolaren Mengen vorliegen.

**12.** Arzneimittel nach Anspruch 11, wobei eines der Enantiomeren einen relativen Anteil von 5 bis 45 Massenprozent am Enantiomerengemisch aufweist.

**13.** Arzneimittel nach einem der Ansprüche 10 bis 12 zur Bekämpfung von Schmerzen.

**14.** Verwendung wenigstens einer Verbindung der allgemeinen Formel I nach Anspruch 1 und/oder von deren Enantiomeren, Diastereomeren, Basen oder Salzen von physiologisch verträglichen Säuren zur Herstellung eines Arzneimittels zur Bekämpfung von Schmerzen.

**Claims**

**1.** Substituted dimethyl-[1-(1-phenyl-cyclohexyl)-piperidin-3-ylmethyl]-amines of the general formula I

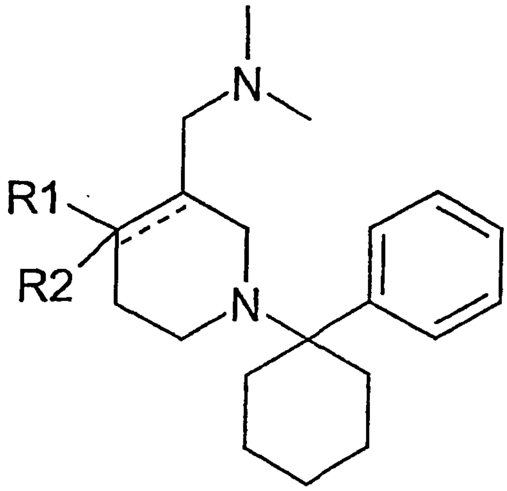

I

wherein

R1 = H, $C_{1-12}$-alkyl (branched, unbranched), vinyl, phenyl (mono- or poly-substituted by $C_{1-5}$-alkyl (branched, unbranched), H, F, C1, Br, OMe, OEt, OPr, OBu, SMe, OH and/or by $CF_3$),
benzyl (mono- or poly-substituted by $C_{1-5}$-alkyl (branched, unbranched), H, F, C1, Br, OMe, OEt, OPr, OBu, SMe, OH and/or by $CF_3$),
phenethyl (mono- or poly-substituted by $C_{1-5}$-alkyl (branched, unbranched), H, F, C1, Br, OMe, OEt, OPr, OBu, SMe, OH and/or by $CF_3$) or
naphthyl (mono- or poly-substituted by $C_{1-5}$-alkyl (branched, unbranched), H, F, Cl, Br, OMe, OEt, OPr, OBu, OBz, SMe, OH and/or by $CF_3$), and
R2 = H, F, Cl, Br, OMe, OEt, OPr, OBu, OBz, SMe, OH, $CF_3$ or bond to the double bond,

and/or their enantiomers, diastereoisomers, bases or salts of physiologically tolerable acids.

**2.** Compounds according to claim 1, **characterised in that** R1 is unbranched $C_{1-8}$-alkyl and R2 has the meaning according to claim 1.

**3.** Compounds according to claim 1, **characterised in that** R1 is vinyl and R2 has the meaning according to claim 1.

**4.** Compounds according to claim 1, **characterised in that** R1 is a phenyl radical substituted by F, Cl, OH or by OMe and R2 has the meaning according to claim 1.

**5.** Compounds according to claim 1, **characterised in that** R1 is benzyl and R2 has the meaning according to claim 1.

**6.** Compounds according to claim 1, **characterised in that** R1 is phenethyl and R2 has the meaning according to claim 1.

**7.** Compounds according to claim 1, **characterised in that** R2 is OH and R1 has the meaning according to claim 1.

**8.** Compounds according to claim 1:

3-dimethylaminomethyl-4-methyl-1-(1-phenylcyclohexyl)-piperidin-4-ol or the corresponding dihydrochloride
3-dimethylaminomethyl-4-ethyl-1-(1-phenyl-cyclohexyl)-piperidin-4-ol or the corresponding dihydrochloride
3-dimethylaminomethyl-1-(1-phenyl-cyclohexyl)-4-vinylpiperidin-4-ol or the corresponding dihydrochloride
4-butyl-3-dimethylaminomethyl-1-(1-phenyl-cyclohexyl)-piperidin-4-ol or the corresponding dihydrochloride
3-dimethylaminomethyl-4-octyl-1-(1-phenyl-cyclohexyl)-piperidin-4-ol or the corresponding dihydrochloride
3-dimethylaminomethyl-4-(3-methoxy-phenyl)-1-(1-phenyl-cyclohexyl)-piperidin-4-ol or the corresponding dihydrochloride
3-dimethylaminomethyl-4-(2-fluoro-phenyl)-1-(1-phenylcyclohexyl)-piperidin-4-ol or the corresponding dihydrochloride
4-(3-chloro-phenyl)-3-dimethylaminomethyl-1-(1-phenylcyclohexyl)-piperidin-4-ol or the corresponding dihydrochloride
4-benzyl-3-dimethylaminomethyl-1-(1-phenylcyclohexyl)-piperidin-4-ol or the corresponding dihydrochloride
3-dimethylaminomethyl-4-phenethyl-1-(1-phenylcyclohexyl)-piperidin-4-ol or the corresponding dihydrochloride
3-dimethylaminomethyl-4-(3-hydroxy-phenyl)-1-(1-phenyl-cyclohexyl)-piperidin-4-ol or the corresponding dihydrochloride.

**9.** Process for the preparation of substituted dimethyl-[1-(1-phenyl-cyclohexyl)-piperidin-3-ylmethyl)amines of the general formula I according to claim 1, wherein cyclohexanone (formula **II**) is reacted with 1,4-dioxa-8-aza-spiro[4.5]decane (formula **III)** in the presence of titanium tetrachloride to form the enamine of formula **IV**

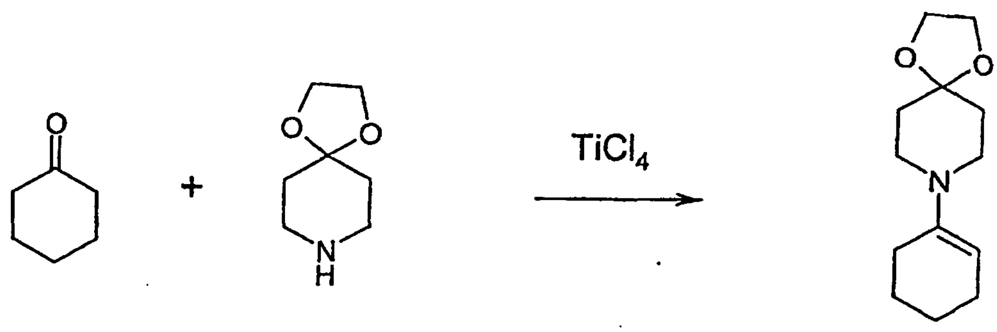


**II** **III** **IV**

which is reacted further with phenylmagnesium chloride (formula V) in the presence of trimethylchlorosilane to form the amine of formula **VI**

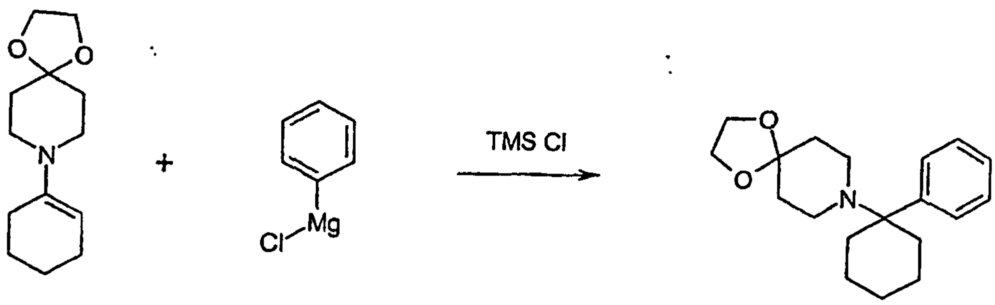


**IV** **V** **VI**

the resulting amine of formula **VI** is then hydrolysed and precipitated in the form of the hydrochloride of formula VII

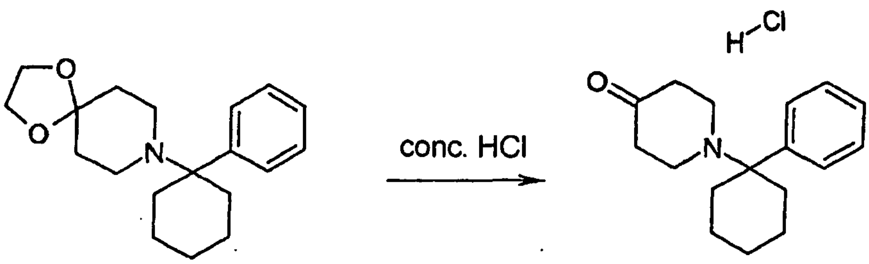


VI VII

which is reacted further with a variant of the Eschenmoser salt according to formula **VIII** to form the Mannich base of formula **IX**

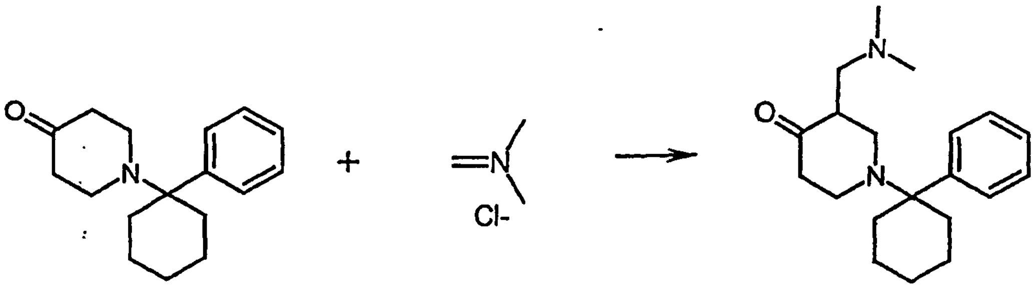

VII VIII IX

following which reaction of the free base according to formula **IX** with a Grignard reagent of formula **X,** which has the organic radical R1, is carried out to form the compounds of formula **XI**

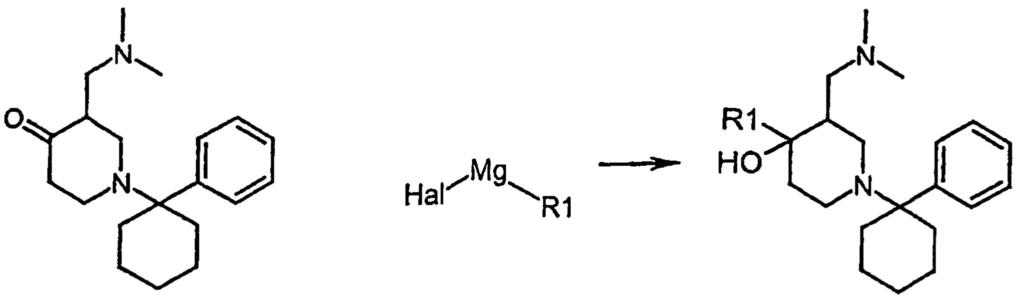

IX X XI

which are purified by conventional methods and isolated in the form of salts of physiologically tolerable acids, wherein

- compounds of formula **XII** are obtained by reacting compounds of formula **XI** with reagents that replace the OH group in the 4-position of the compounds of formula **XI** by the above-defined radical R2, with the exception of the OH group, especially halogenated hydrocarbons, ethers, esters, ureas, amides, carbonates and related compounds

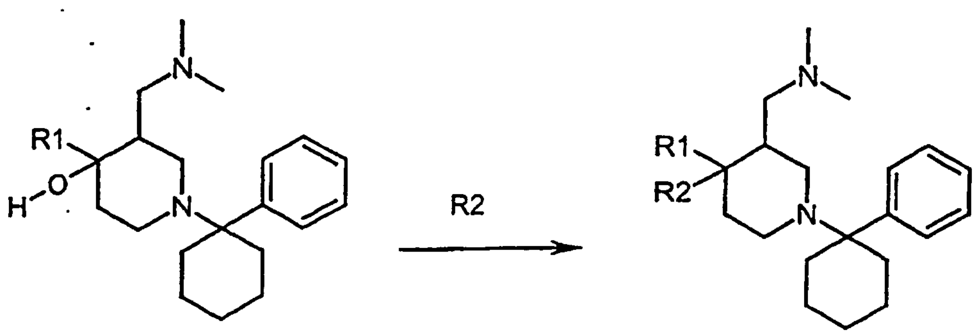


XI XII

• compounds of formula **XIII** are obtained by dehydrating compounds of formula **XI**

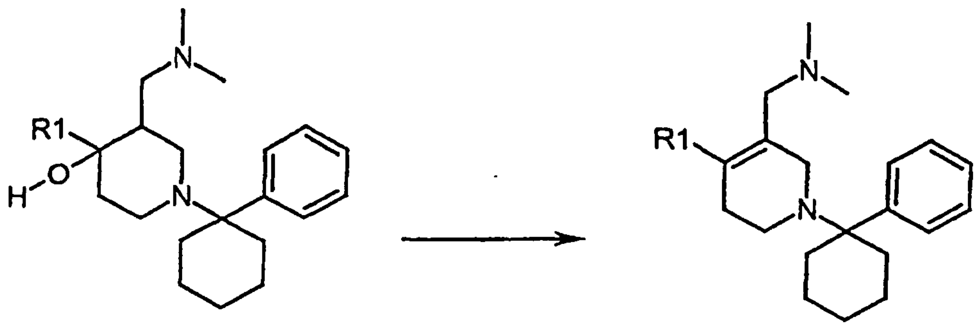

XI XIII

and

• compounds of formula **XIV** are obtained by reducing compounds of formula **XIII** with hydrogen

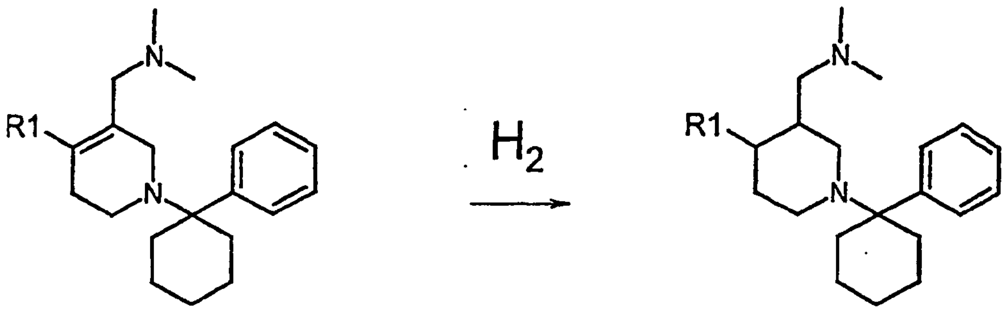


XIII XIV

**10.** Medicament containing as active ingredient at least one compound of the general formula I according to claim 1 and/or its enantiomers, diastereoisomers, bases or salts of physiologically tolerable acids.

**11.** Medicament according to claim 10 containing as active ingredient a mixture of the enantiomers of a compound of the general formula I according to claim 1, wherein the two enantiomers are not present in equimolar amounts.

**12.** Medicament according to claim 11, wherein one of the enantiomers has a relative content of from 5 to 45 wt.% in the enantiomeric mixture.

**13.** Medicament according to any one of claims 10 to 12 for controlling pain.

**14.** Use of at least one compound of the general formula **I** according to claim 1 and/or its enantiomers, diastereoisomers, bases or salts of physiologically tolerable acids in the manufacture of a medicament for controlling pain.

## Revendications

**1.** Diméthyl[1-(1-phénylcyclohexyl)pipéridine-3-yl-méthyl]amines substituées, de formule générale **I**

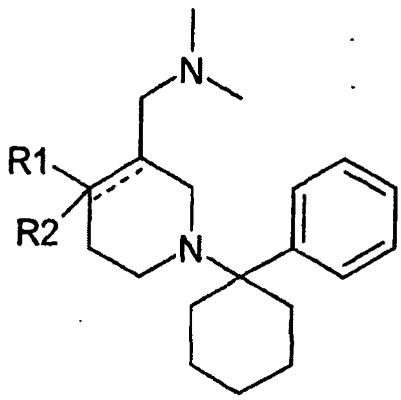

**I**

dans laquelle
R1 représente H, un reste alkyle en $C_1$ à $C_{12}$ (ramifié, non ramifié), vinyle, phényle (substitué une ou plusieurs fois avec un radical alkyle en $C_1$ à $C_5$ (ramifié, non ramifié), H, F, Cl, Br, OMe, OEt, OPr, OBu, SMe, OH et/ou $CF_3$),
un reste benzyle (substitué une ou plusieurs fois avec un radical alkyle en $C_1$ à $C_5$ (ramifié, non ramifié), H, F, Cl, Br, OMe, OEt, OPr, OBu, SMe, OH et/ou $CF_3$),
un reste phénéthyle (substitué une ou plusieurs fois avec un radical alkyle en $C_1$ à $C_5$ (ramifié, non ramifié), H, F, Cl, Br, OMe, OEt, OPr, OBu, SMe, OH et/ou $CF_3$), ou bien
un reste naphtyle (substitué une ou plusieurs fois avec un radical alkyle en $C_1$ à $C_5$ (ramifié, non ramifié), H, F, Cl, Br, OMe, OEt, OPr, OBu, OBz, SMe, OH et/ou $CF_3$), et
R2 représente H, F, Cl, Br, OMe, OEt, OPr, OBu, OBz, SMe, OH, $CF_3$ ou une liaison relative à la double liaison,
et/ou leurs énantiomères, leurs diastéréoisomères, leurs bases ou leurs sels d'acides acceptables du point de vue physiologique.

**2.** Composés suivant la revendication 1, **caractérisés en ce que** R1 est un reste alkyle en $C_1$ à $C_8$ non ramifié et R2 a la définition suivant la revendication 1.

**3.** Composés suivant la revendication 1, **caractérisés en ce que** R1 est un reste vinyle et R2 a la définition suivant la revendication 1.

**4.** Composés suivant la revendication 1, **caractérisés en ce que** R1 est un reste phényle substitué avec F, Cl, OH ou OMe et R2 a la définition selon la revendication 1 .

**5.** Composés suivant la revendication 1, **caractérisés en ce que** R1 est un reste benzyle et R2 a la définition selon la revendication 1.

**6.** Composés suivant la revendication 1, **caractérisés en ce que** R1 est un reste phénéthyle et R2 a la définition selon la revendication 1.

7. Composés suivant la revendication 1, **caractérisés en ce que** R2 est le groupe OH et R1 a la définition selon la revendication 1.

8. Composés suivant la revendication 1, à savoir,
3-diméthylaminométhyl-4-méthyl-1-(1-phénylcyclohexyl)-pipéridine-4-ol ou le dichlorhydrate correspondant
3-diméthylaminométhyl-4-éthyl-1-(1-phénylcyclohexyl)-pipéridine-4-ol ou le dichlorhydrate correspondant
3-diméthylaminométhyl-1-(1-phénylcyclohexyl)-4-vinylpipéridine-4-ol ou le dichlorhydrate correspondant
4-butyl-3-diméthylaminométhyl-1-(1-phénylcyclohexyl)-pipéridine-4-ol ou le dichlorhydrate correspondant
3-diméthylaminométhyl-4-octyl-1-(1-phénylcyclohexyl)-pipéridine-4-ol ou le dichlorhydrate correspondant
3-diméthylaminométhyl-4-(3-méthoxyphényl)-1-(1-phénylcyclohexyl)pipéridine-4-ol ou le dichlorhydrate correspondant
3-diméthylaminométhyl-4-(2-fluorophényl)-1-(1-phénylcyclohexyl)pipéridine-4-ol ou le dichlorhydrate correspondant
4-(3-chlorophényl)-3-diméthylaminométhyl-1-(1-phénylcyclohexyl)pipéridine-4-ol ou le dichlorhydrate correspondant
4-benzyl-3-diméthylaminométhyl-1-(1-phénylcyclohexyl)-pipéridine-4-ol ou le dichlorhydrate correspondant
3-diméthylaminométhyl-4-phénéthyl-1-(1-phénylcyclohexyl)pipéridine-4-ol ou le dichlorhydrate correspondant
3-diméthylaminométhyl-4-(3-hydroxyphényl)-1-(1-phénylcyclohexyl)pipéridine-4-ol ou le dichlorhydrate correspondant.

9. Procédé de production de diméthyl[1-(1-phénylcyclohexyl)pipéridine-3-ylméthyl]amines substituées de formule générale I suivant la revendication 1, dans lequel on fait réagir la cyclohexanone (formule II) avec le 1,4-dioxa-8-aza-spiro[4.5]décane (formule III) en présence de tétrachlorure de titane pour obtenir l'énamine de formule IV

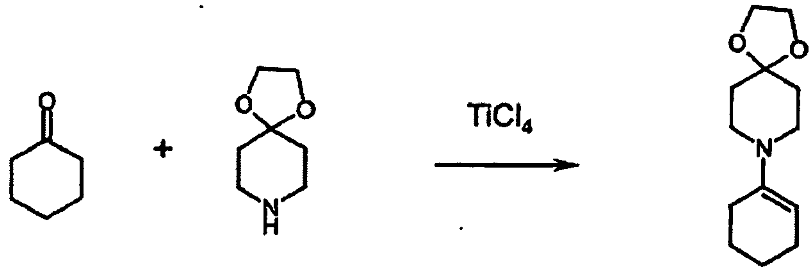


II III IV

qu'on transforme ensuite avec le chlorure de phénylmagnésium (formule **V)** en présence de triméthylchlorosilane en l'amine de formule **VI**

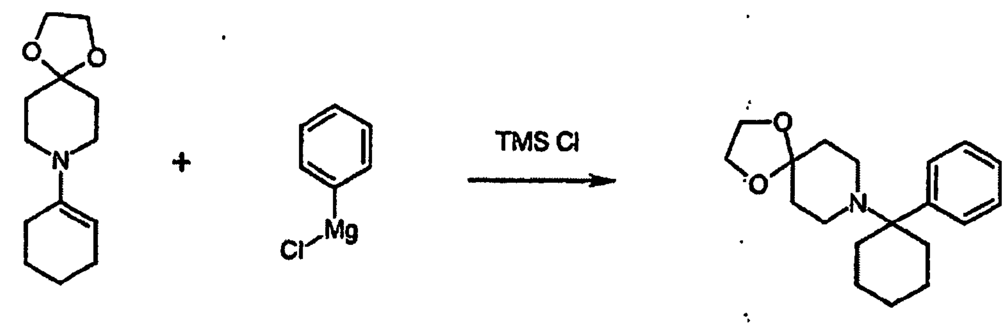


IV V VI

puis on hydrolyse l'amine obtenue de formule VI et on la précipite sous forme de chlorhydrate de formule VII

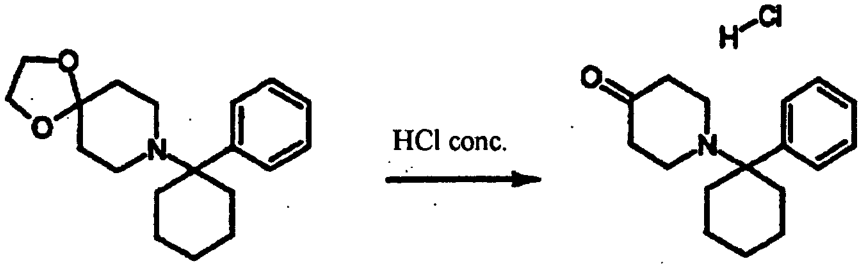


VI VII

qu'on transforme ensuite avec une variante du sel d'Eschenmoser selon la formule **VIII** en la base de Mannich de formule **IX**

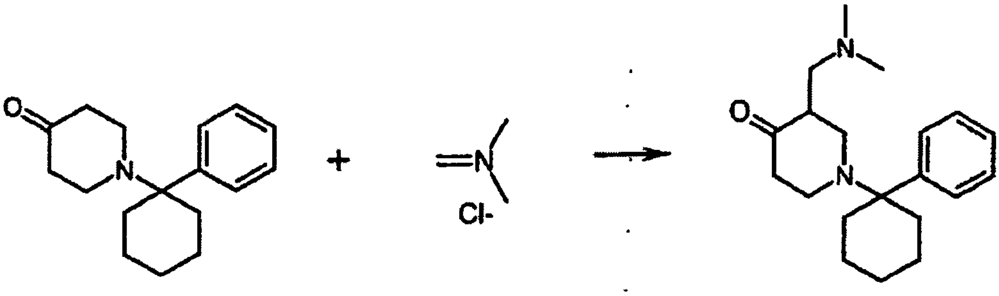

VII VIII IX

après quoi on conduit la réaction de la base libre de formule **IX** avec un réactif de Grignard de formule **X,** qui présente le reste organique R1, pour obtenir les composés de formule **XI**

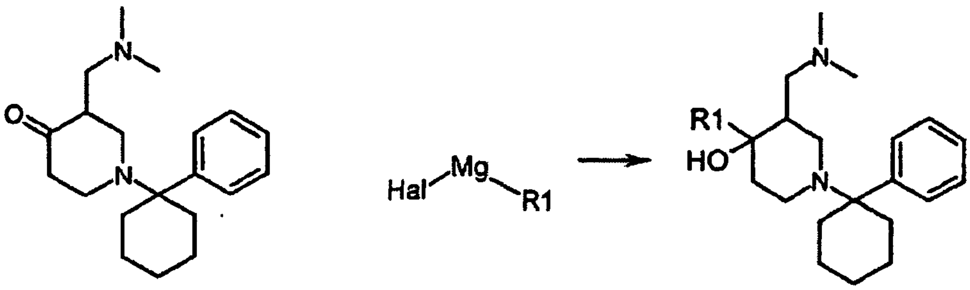

IX X XI

qu'on purifie selon des modes opératoires usuels et qu'on isole sous forme de sels d'acides acceptables du point de vue physiologique, de sorte que

- on obtient des composés de formule **XII** en faisant réagir des composés de formule **XI** avec des réactifs qui remplacent le groupe OH dans la position 4 des composés de formule **XI** par le reste R2 défini ci-dessus, excepté le groupe OH, en particulier des hydrocarbures halogénés, des éthers, des esters, des urées, des amides, des carbonates et des composés apparentés

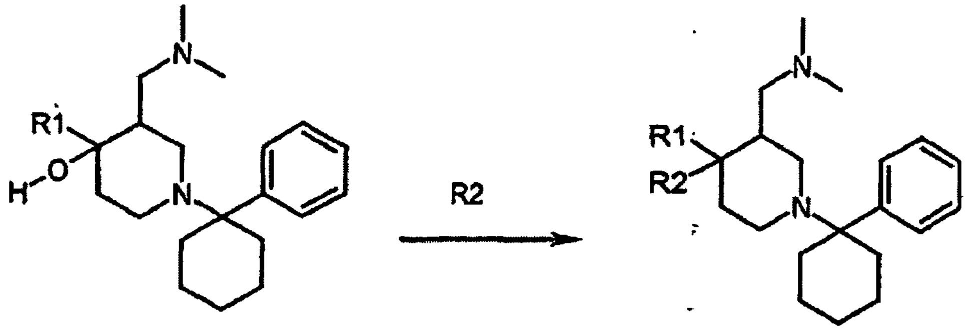


XI XII

• on obtient des composés de formule **XIII** en déshydratant des composés de formule **XI**

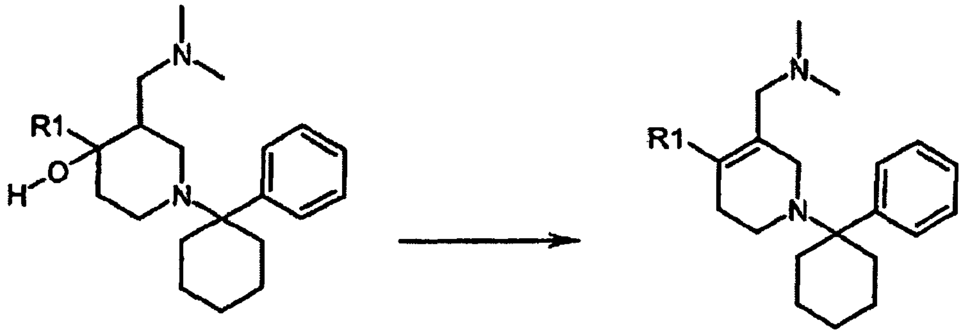

XI XIII

et

• on obtient des composés de formule **XIV** en réduisant des composés de formule **XIII** avec l'hydrogène

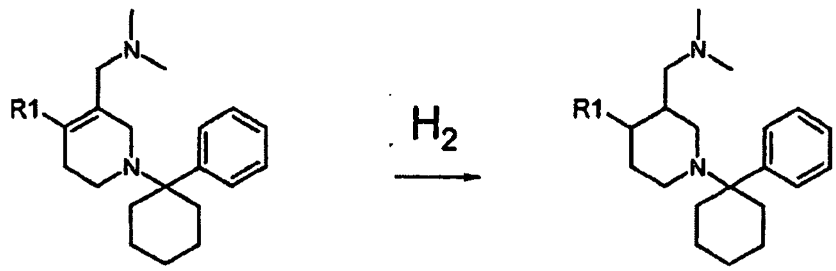


XIII XIV

**10.** Médicament contenant comme substance active au moins un composé de formule générale I suivant la revendication 1 et/ou ses énantiomères, ses diastéréoisomères, ses bases ou ses sels d'acides acceptables du point de vue physiologique.

**11.** Médicament suivant la revendication 10, contenant comme substance active un mélange des énantiomères d'un composé de formule générale I suivant la revendication 1, les deux énantiomères n'étant pas présents en quantités équimolaires.

**12.** Médicament suivant la revendication 11, dans lequel l'un des énantiomères est en proportion de 5 à 45 pour cent

en masse relativement au mélange d'énantiomères.

**13.** Médicament suivant l'une des revendications 10 à 12, destiné à combattre des douleurs.

**14.** Utilisation d'au moins un composé de formule générale I suivant la revendication 1 et/ou de ses énantiomères, ses diastéréoisomères, ses bases ou ses sels d'acides acceptables du point de vue physiologique pour la préparation d'un médicament destiné à combattre des douleurs.